# EUROPÄISCHE PATENTANMELDUNG

(11) **EP 4 062 946 A2**
(43) Veröffentlichungstag der Anmeldung: **28.09.2022**
(21) Anmeldenummer: 22156928.8
(22) Anmeldetag: 16.02.2022
(51) Int. Cl.: A61L 2/10, A47B 88/919

(54) **MÖBEL MIT EINEM MÖBELKORPUS UND EINEM BEWEGLICHEN MÖBELTEIL**

(30) Priorität: 24.03.2021 DE 202021101546 U
(71) Anmelder: Grass GmbH, 6973 Höchst (AT)
(72) Erfinder: Mittwoch, Stephan, 6890 Lustenau (AT); Grabher, Günter, 6972 Fußach (AT)
(74) Vertreter: Otten, Roth, Dobler & Partner mbB Patentanwälte

(57) **Zusammenfassung**

Es wird ein Möbel (1) mit einem Möbelkorpus (2) und einem beweglichen Möbelteil (4, 23, 26) vorgeschlagen, wobei das bewegliche Möbelteil (4, 23, 26) relativ zum Möbelkorpus (2) beweglich ist, wobei am Möbelkorpus (2) und/oder an dem beweglichen Möbelteil (4, 23, 26) eine Beleuchtungsanordnung (15) angeordnet ist, wobei die Beleuchtungsanordnung (15) ein Leuchtmittel (14) umfasst. Erfindungsgemäß strahlt das Leuchtmittel (14) ein funktionales Licht in einen Innenraum (30) des Möbelkorpus (2) und/oder in einen Innenraum (31) des beweglichen Möbelteils (4) ab, wobei das funktionale Licht Luft und/oder Oberflächen und/oder Gegenständen in einen Innenraum (30) des Möbelkorpus (2) und/oder einen Innenraum (31) des beweglichen Möbelteils (4) entkeimt und/oder erwärmt.

## Beschreibung

### Stand der Technik

Aus dem Stand der Technik sind Beleuchtungsanordnungen für Möbel bekannt, wobei mit den Beleuchtungsanordnungen der Inhalt bzw. ein Innenraum von beweglichen Möbelteilen, wie z.B. Schubladen, oder des Möbelkorpus beleuchtet werden soll.

Mit der Beleuchtungsanordnung soll es erleichtert werden, Gegenstände innerhalb des beweglichen Möbelteils oder Möbelkorpus zu finden und/oder die Ästhetik bzw. das äußere Erscheinungsbild des Möbels verbessert werden.

### Aufgabe und Vorteile der Erfindung

Aufgabe der Erfindung ist es, eine verbesserte Beleuchtungsanordnung für ein Möbel bereitzustellen, insbesondere im Hinblick auf eine erweiterte Funktionalität der Beleuchtung des Innenraums des Möbels.

Die Aufgabe wird durch die Merkmale der unabhängigen Ansprüche gelöst.

Vorteilhafte und zweckmäßige Ausführungsformen, Varianten und Weiterbildungen der Erfindung sind in den abhängigen Ansprüchen ausgeführt.

Die Erfindung geht aus von einem Möbel mit einem Möbelkorpus und einem beweglichen Möbelteil, wobei das bewegliche Möbelteil relativ zum Möbelkorpus beweglich ist, wobei am Möbelkorpus und/oder an dem beweglichen Möbelteil eine Beleuchtungsanordnung angeordnet ist, wobei die Beleuchtungsanordnung ein Leuchtmittel umfasst.

Das Leuchtmittel kann neben dem Möbelkorpus und dem beweglichen Möbelteil auch an den Beschlägen und/oder den Führungsmittel angeordnet werden. Auch kann ein Leuchtmittel am Möbelkorpus und/oder dem beweglichen Möbelteil und ein andere Leuchtmittel am Beschlag und/oder Führungsmittel angeordnet sein.

Vorteilhafterweise sind Beschläge und/oder Führungsmittel am Möbel vorgesehen, so dass mittels den Beschlägen und/oder den Führungsmitteln das bewegliche Möbelteil relativ zum Möbelkorpus beweglich ist. Vorzugsweise sind Führungsmittel bzw. Führungsschienen vorgesehen, wenn das bewegliche Möbelteil eine Schublade ist und bevorzugterweise sind Beschläge vorgesehen, wenn das bewegliche Möbelteil eine Klappe, wie z.B. eine Oberklappe, oder eine Türe, wie z.B. eine Schranktüre, ist.

Der Kern der Erfindung ist darin zu sehen, dass das Leuchtmittel ein funktionales Licht in einen Innenraum des Möbelkorpus und/oder in einen Innenraum des beweglichen Möbelteils abstrahlt, wobei das funktionale Licht Luft und/oder Oberflächen und/oder Gegenständen in einen Innenraum des Möbelkorpus und/oder einen Innenraum des beweglichen Möbelteils entkeimt und/oder erwärmt.

Das funktionale Licht ist derart ausgebildet, dass beim Entkeimen die biologischen Mikroorganismen, wie z.B. Viren und/oder Bakterien, die sich auf Oberflächen und in der Luft befinden abgetötet bzw. zerstört werden. Damit werden die Oberflächen und/oder die Luft desinfiziert.

Das funktionale Licht ist derart ausgebildet, dass beim Erwärmen die Luft und die Gegenstände auf eine Temperatur zwischen 20° bis 100°C, insbesondere 20° bis 50°C, 30° bis 50°C, 35° bis 45°C erwärmt werden.

Bevorzugterweise umfasst das Leuchtmittel mehrere Leuchtdioden, wobei vorzugsweise eine Vielzahl von Leuchtdioden an einem gemeinsamen flexiblen oder starren Träger, beispielsweise an einem Band oder Schlauch, nah nebeneinander angeordnet sind.

Vorteilhafterweise ist das Leuchtmittel der Beleuchtungsanordnung so im Möbelkorpus und/oder am beweglichen Möbelteil und/oder an den Beschlägen und/oder an den Führungsmitteln angeordnet, dass vorzugsweise das komplette Volumen des Innenraums des Möbelkorpus und/oder des beweglichen Möbelteils bestrahlt wird. Das Leuchtmittel kann dafür nur am Möbelkorpus, nur am beweglichen Möbelteil oder die Leuchtmittel können am Möbelkorpus und dem beweglichen Möbelteil angeordnet sein.

Weiter wird vorzugsweise auch ein Gegenstand bzw. werden die Gegenstände, welche sich im Innenraum des Möbels befinden, durch das funktionale Licht beleuchtet bzw. wirkt das Licht auf die Gegenstände. Bevorzugt ist, dass alle Oberflächen des Gegenstandes bzw. der Gegenstände durch das funktionale Licht beleuchtet werden.

Beim Beleuchten des Innenraums des Möbelkorpus und/oder des beweglichen Möbelteils mit funktionalen Licht ist auch bevorzugterweise die Luft im Innenraum des Möbels entkeimbar und/oder erwärmbar. Auch sind die Oberflächen des Möbels und/oder des beweglichen Möbelteils, die zum Innennraum des Möbels ausgerichtet sind, durch das funktionale Licht erwärmbar und/oder entkeimbar.

Bevorzugterweise sind die Oberflächen des Möbelkorpus, beispielsweise die Korpusseitenwände, die Korpusrückwand, der Korpusboden und die Korpusoberseite entkeimbar und/oder erwärmbar. Ist das bewegliche Möbelteil eine Platte bzw. eine Oberklappe oder eine Schranktür, dann ist die Seite, die zum Innenraum des Möbelkorpus ausgerichtet ist entkeimbar. Ist das bewegliche Möbelteil eine Schublade, dann sind vorzugsweise alle Oberflächen der Schubladenseitenwände, des Schubladenbodens und der Schubladenrückwand, also alle Oberflächen, die sich im Innenraum des Möbelkorpus in einem geschlossenen Zustand des Möbels befinden, entkeimbar und/oder erwärmbar durch das funktionale Licht. Nur die Seite der Schubladenfront, welche nach außen gerichtet ist, also nicht zum Innenraum des Möbelkorpus gerichtet ist, ist durch das funktionale Licht nicht entkeimbar und/oder erwärmbar. Vorteilhaft erreichen die Lichtstrahlen des funktionalen Lichts auch kleine Vertiefungen auf der Oberfläche.

Von Vorteil ist, dass das funktionale Licht, welches das Leuchtmittel abstrahlt, im UVC-Bereich liegt, insbesondere eine Wellenlänge zwischen 200-270 nm aufweist.

Die UVC-Strahlung kann Luft und/oder Oberflächen entkeimen. Vorteilhafterweise werden durch das funktionale Licht im UVC-Bereich die Luft und/oder die Oberflächen des Möbelkorpus und/oder des beweglichen Möbelteils, welche sich im Innenraum des Möbels befinden, entkeimt. Weiter können auch Gegenständen im Innenraum des Möbelkorpus und/oder des beweglichen Möbelteils, z.B. bei Schubladen, durch das funktionale Licht im UVC-Bereich entkeimt werden.

Die Wirkung der UVC-Strahlung hängt von der Dosis der UVC-Strahlung ab, welche sich aus der Intensität und der Bestrahlungszeit ergibt. Um Beispielsweise E-Coli Bakterien sicher zu zerstören bzw. abzutöten, wird eine Dosis von ca. 7000 mW*s/cm² benötigt.

Vorzugsweise weist die überwiegende Zahl bzw. insbesondere jede Leuchtdiode der Leuchtmittel eine Leistung von 20-50 mW auf. Vorteilhafterweise sind viele Leuchtdioden vorgesehen, wobei die Leuchtdioden bevorzugterweise nah beieinander im Innenraum des Möbelkorpus und/oder des beweglichen Möbelteils angeordnet sind, so dass mehrere Leuchtdioden dieselbe Fläche bestrahlen. Damit ist die Intensität pro Fläche und damit die Wirksamkeit der biologischen Wirkung des funktionalen Lichts im UVC-Bereich erhöhbar.

Um die gewünschte entkeimende Wirkung der UVC-Strahlung zu erhalten, ist es vorteilhaft, wenn das Leuchtmittel bzw. die Leuchtdioden für mehrere Minuten, vorzugsweise zwischen 2 und 5 Minuten die Oberfläche des Möbels im Innenraum und/oder Gegenstände im Innenraum des Möbels bzw. die Luft im Innenraum des Möbels bestrahlen.

Eine weitere vorteilhafte Variante besteht darin, dass das funktionale Licht des Leuchtmittels im Infrarotbereich liegt.

Bevorzugterweise kann mit Infrarotstrahlung Wärme im Innenraum des Möbels erzeugt werden, womit Gegenstände wie z.B. Lebensmittel im Innenraum des Möbelkorpus und/oder des beweglichen Möbelteils erwärmt werden können. Auch kann durch das funktionale Licht im Infrarotbereich Oberflächen und/oder Luft im Innenraum des Möbelkorpus und/oder des beweglichen Möbelteils erwärmt werden.

Der Infrarotbereich des funktionalen Lichts liegt vorzugsweise bei 700 bis 1000 nm. Das Leuchtmittel, welches funktionales Licht im Infrarotbereich erzeugt, umfasst vorzugsweise Leuchtdioden bzw. Infrarotdioden. Die Leuchtmittel sind vorteilhafterweise an Flächen des Möbelkorpus und/oder des beweglichen Möbelteils angeordnet, welche zum Innenraum des Möbels bzw. des Möbelkorpus ausgerichtet sind.

Von Vorteil ist, dass die Beleuchtungseinrichtung eine Sensoreinheit und eine Kontrolleinheit umfasst, wobei die Sensoreinheit einen Bewegungszustand des beweglichen Möbelteils erfasst und wobei die Kontrolleinheit ausgebildet ist, abhängig von einem mit der Sensoreinheit erfassten Bewegungszustand des beweglichen Möbelteils das Leuchtmittel ein- und/oder auszuschalten, so dass das Leuchtmittel ausschließlich bei einem geschlossen Zustand des Möbels einschaltbar ist.

Die Kontrolleinheit umfasst vorzugsweise eine Rechnereinheit mit einer programmierbaren Speichereinheit bzw. Steuereinheit, dabei kommunizieren die Kontrolleinheit und die Sensoreinheit zumindest zeitweise miteinander. Weiter kann die Kontrolleinheit auch eine Schaltanordung bzw. einen Schalter aufweisen, z.B. einen manuell oder automatisch schaltbaren Schalter, mit welchem die Stromzufuhr zum Leuchtmittel gezielt unterbrechbar oder herstellbar ist, indem der Schalter einen offenen oder geschlossenen Schaltzustand einnimmt. Die Kontrolleinheit und/oder die Sensoreinheit sind vorzugsweise an unterschiedlichen Stellen des beweglichen Möbelteils und/oder des Möbelkorpus anbringbar. Die Kontrolleinheit ist vorzugsweise am gleichen Teil des Möbels angebracht an welchem auch das Leuchtmittel angeordnet ist. Ist beispielsweise das Leuchtmittel am Möbelkorpus angeordnet, ist bevorzugterweise auch die Kontrolleinheit am Möbelkorpus angeordnet.

Auf die vorgenannte Weise kann flexibel und zuverlässig das funktionale Licht ein- und ausgeschaltet werden. Um das Beleuchten mit funktionalen Licht auf einen geschlossenen Zustand des Möbels bzw. des beweglichen Möbelteils einzuschränken, wird vorteilhaft bzw. individuell auf unterschiedliche Detektionsprinzipien zurückgegriffen. Demgemäß kommen verschiedene Sensoren der modernen Detektionstechnik zum Einsatz. Diese weisen gegenüber anderen Techniken jeweils dazugehörige bzw. spezifische Vorteile auf. Allen gemeinsam sind die technisch hoch präzise Wirkweise, die hohe Zuverlässigkeit, eine hohe Wirtschaftlichkeit und/oder eine hohe Robustheit. Außerdem ist der benötigte Bauraum vergleichsweise gering.

Die Sensoreinheit kann unterschiedliche Arten von insbesondere elektrischen Sensoren umfassen, wie z.B. optische Sensoren, piezoelektrische Sensoren, magnetische Sensoren, induktive Sensoren und/oder kapazitive Sensoren. Die Sensoren erfassen bevorzugterweise eine Bewegung bzw. Beschleunigung des beweglichen Möbelteils.

Die Sensoreinheit kommuniziert vorteilhafterweise, dass das bewegliche Möbelteil im geschlossenen Zustand ist an die Kontrolleinheit, so dass die Kontrolleinheit, z.B. durch Schließen eines Schalters, die Leuchtmittel ein- bzw. anschaltet. Wenn das bewegliche Möbelteil aus dem geschlossenen Zustand in den geöffneten Zustand bewegt wird, wird dies von der Sensoreinheit erkannt und an die Kontrolleinheit übermittelt, so dass die Kontrolleinheit, z.B. durch Öffnen des Schalters, das Leuchtmittel ausschaltet.

Es ist auch möglich, dass die Kontrolleinheit die Sensoreinheit umfasst, so dass die Kontrolleinheit einen Bewegungszustand des beweglichen Möbelteils detektiert, wie z.B. bei einer Reedeinheit, welche einen Reedschalter und einen Magneten umfasst. Wird eine Reedeinheit als Kontrolleinheit verwendet, so ist an einem Möbelteil der Magnet und am anderen Möbelteil der Reedschalter angeordnet. Ist beispielsweise der Reedschalter am beweglichen Möbelteil angeordnet, dann ist der Magnet am Möbelkorpus angeordnet.

Ebenfalls von Vorteil ist, dass die Kontrolleinheit so ausgebildet ist, dass das Leuchtmittel, nur im geschlossenen Zustand des Möbels, von einer Bedienperson aktivierbar ist.

Durch energiereiche Strahlung, wie z.B. UVC-Strahlung, werden Materialien mit der Zeit zersetzt, insbesondere organische Materialien, wie z.B. Kunststoff. Aus diesem Grund ist es bevorzugt, wenn nicht bei jedem Schließen des beweglichen Möbelteils das funktionale Licht eingeschaltet wird. Auch kann dadurch vorteilhafterweise Energie gespart werden.

Dadurch, dass die Bedienperson das funktionale Licht des Leuchtmittels aktiviert, ist gewährleistet, dass das funktionale Licht nicht bei jedem Schließen des beweglichen Möbelteils eingeschaltet wird. Außerdem ist es erwünscht, dass die Bedienperson selbst beeinflussen kann, wann die Oberflächen und/oder die Luft im Innenraum des Möbels entkeimt und/oder erwärmt werden soll.

Vorteilhafterweise kann die Bedienperson das Leuchtmittel durch einen mechanischen Schalter, welcher am Möbelkorpus und/oder am beweglichen Möbelteil angeordnet ist, aktivieren. Dabei kann ein direkt betätigbarer Schalter, wie beispielsweise ein Druckschalter, ein Kippschalter, ein Tastschalter oder dergleichen verwendet werden. Auch die Verwendung eines indirekt betätigbaren Schalters, wie beispielsweise ein Funkschalter, ist möglich. Bei dem Funkschalter kann das Aktivieren des Funkschalters durch die Bedienperson mittels einer Fernbedienung erfolgen.

Dabei kann das Leuchtmittel nur durch die Bedienperson aktiviert bzw. eingeschaltet werden, wenn die Sensoreinheit der Kontrolleinheit kommuniziert hat, dass das bewegliche Möbelteil geschlossen ist. So kann erst, wenn z.B. der elektrische Schalter der Kontrolleinheit geschlossen ist, die Bedienperson durch Schalten des mechanischen Schalters die Leuchtmittel aktivieren bzw. den dazugehörigen elektrischen Stromkreis schließen.

Ein weiterer Aspekt der Erfindung ist, dass die Kontrolleinheit ausgebildet ist, das Leuchtmittel nach dem Aktivieren durch eine Bedienperson, für eine vorgebbare Zeitspanne einzuschalten.

Die vorgebbare Zeitspanne beträgt vorzugsweise 1 bis 5 Minuten, insbesondere 1 bis 3 Minuten. Die vorgebbare Zeitspanne ist vorzugsweise durch ein Zeitglied einstellbar. Das Zeitglied kommuniziert vorteilhafterweise mit der Kontrolleinheit. Mit dem Zeitglied kann eingestellt werden, dass das Leuchtmittel für eine bestimmten Zeit, insbesondere 1 bis 3 Minuten, einschaltbar ist. Das Zeitglied kann am Möbelkorpus und/oder dem beweglichen Möbelteil angeordnet sein. Das Zeitglied kann auch an dem Leuchtmittel bzw. an dem Träger wie z.B. dem Band oder Schlauch, an dem die Leuchtdioden angeordnet sind, befestigt sein.

Im Regelfall wird dies in der Elektrotechnik mit einem IC-Timerbaustein realisiert. Dies ist ein aktives Bauteil, welches auf eine externe Stromquelle angewiesen ist und welches über einen Mikrokontroller ansteuerbar und softwareseitig variabel einstellbar ist. Ein passives Zeitglied hat den Vorteil, dass es keinen Anschluss an eine externe Stromquelle benötigt, um die gewünschte Funktion umzusetzen. Hierfür wird diese Funktion einmalig dimensioniert und ist dann fest parametriert oder gegebenenfalls über einen Potentiometer einstellbar.

Weiter kann an der Außenseite des Möbelkorpus und/oder des beweglichen Möbelteils eine Anzeige angeordnet sein, die die verbleibende oder abgelaufene Beleuchtungszeit der Bedienperson anzeigt. Die Anzeige kommuniziert vorzugsweise mit der Kontrolleinheit, welche mit dem Zeitglied in Verbindung steht.

Vorteilhafterweise umfasst die Beleuchtungsanordnung einen Energiespeicher, wobei der Energiespeicher das Leuchtmittel und/oder die Kontrolleinheit mit elektrischer Energie versorgt.

Der Energiespeicher stellt vorteilhafterweise eine Niedervolt-Spannung, insbesondere einer Niedervoltarbeitsspannung von unter 50 Volt bereit.

Der Energiespeicher ist vorzugsweise ein Akku, welche an dem beweglichen Möbelteil und/oder an dem Möbelkorpus, insbesondere einer Rückwand des Möbelkorpus und/oder des beweglichen Möbelteils, angeordnet ist. Die Versorgung des Leuchtmittels und/oder der Kontrolleinheit mit elektrischen Strom kann auch alternativ über eine feste Verbindung, mit z.B. dem Stromnetz, erfolgen.

Der Energiespeicher weist bevorzugterweise zumindest eine Ansteckstelle, vorzugsweise zwei Ansteckstellen, auf. Die eine Ansteckstelle ist bevorzugterweise geeignet, um den Energiespeicher zu laden und die anderen Ansteckstelle, um den Energiespeicher mittels einer elektrischen Leitung mit den Leuchtmitteln und/oder Kontrolleinheit mittels einer Steckverbindung zu verbinden. Die Ansteckstellen sind vorzugsweise für standardisierte Stecker, wie z.B. mini USB und/oder micro USB, geeignet. Weiter ist der Energiespeicher vorteilhafterweise abnehmbar am beweglichen Möbelteil und/oder Möbelkorpus, insbesondere einer Rückwand des beweglichen Möbelteils und/oder Möbelkorpus, angeordnet.

Vorteilhaft ist, dass die Kontrolleinheit ausgebildet ist, bei einer durch die Sensoreinheit erfassten Öffnungsbewegung des beweglichen Möbelteils das Leuchtmittel auszuschalten.

Wird das bewegliche Möbelteil geöffnet, während das Leuchtmittel aktiviert ist bzw. leuchtet, kommuniziert die Sensoreinheit die Öffnungsbewegung an die Kontrolleinheit, die beispielsweise durch Öffnen eines Schalters das Leuchtmittel ausschaltet bzw. den Stromkreis unterbricht. Umfasst die Kontrolleinheit die Sensoreinheit, wie z.B. beim Reedschalter, wird durch die Öffnungsbewegung des beweglichen Möbelteils der Reedschalter vom Magneten weg bewegt, wodurch der Reedschalter sich öffnet und den Stromkreis unterbricht und somit das Leuchtmittel ausschaltet. Von Vorteil ist, dass durch das Ausschalten des Leuchtmittels beim Öffnen des beweglichen Möbelteils die Bedienperson, im Besonderen die Augen der Bedienperson, keinen Schaden nehmen durch das funktionale Licht.

Es ist überdies vorteilhaft, dass die Kontrolleinheit ausgebildet ist, einen Sperrmechanismus zu bedienen, wobei der Sperrmechanismus derart ausgebildet ist, das bewegliche Möbelteil, wenn das Leuchtmittel eingeschaltet ist, zu versperren und bei ausgeschalteten Leuchtmittel das bewegliche Möbelteil wieder zu entsperren.

Der Sperrmechanismus kann unterschiedlich ausgestaltet werden, beispielsweise kann das bewegliche Möbelteil durch einen schwenkbaren Haken von der Öffnungsbewegung gehindert werden. Dabei ist vorzugsweise der schwenkbare Haken am Möbelkorpus angeordnet. Der schwenkbare Haken wird vorteilhafterweise nach dem Betätigen des mechanischem Schalters durch die Bedienperson in Richtung des beweglichen Möbelteils verschwenkt und schwenkt nach der durch das Zeitglied vorgebaren Zeitdauer zurück in die Ausgangsposition.

Weiter kann der Sperrmechanismus auch einen höhenverstellbaren Bolzen aufweisen, welcher beim Aktivieren des Leuchtmittels durch die Bedienperson in Richtung des beweglichen Möbelteils verschoben wird. Das bewegliche Möbelteil kann dafür auch eine Ausnehmung aufweisen, so dass der Bolzen in die Ausnehmung am beweglichen Möbelteil eingreift.

Eine weitere Möglichkeit besteht darin, dass beim Aktivieren des Leuchtmittels durch die Bedienperson ein Elektromagnet aktiviert wird, welcher mit einem durch einen Magnet anziehbaren Material wechselwirkt. Das durch den Magneten anziehbare Material ist bevorzugterweise am beweglichen Möbelteil angeordnet.

Von Vorteil ist, dass das bewegliche Möbelteil eine Schublade ist, wobei das Leuchtmittel an einer Schubladenreling und/oder an einer Querreling und/oder an einer Schubladenseitenwand und/oder an einer Zarge und/oder an einem Seitenwandelement und/oder an einer Schubladenrückwand und/oder an einem Schubladenboden und/oder an einer Schubladenfront angeordnet ist.

Die Schubladenseitenwand umfasst vorzugsweise eine Zarge und ein Seitenwandelement.

Da beispielsweise UVC-Strahlung viele transluzente oder transparente Materialien, wie z.B. handelsübliches Glas, nicht durchdringt, strahlt das funktionale Licht vorzugsweise direkt in den Innenraum des Möbelkorpus und/oder der Schublade. Bevorzugterweise sind dafür Ausnehmungen an der Schubladenreling und/oder der Querreling und/oder der Zarge vorhanden, damit das funktionale Licht direkt in den Innenraum strahlen kann. Die Ausnehmungen sind vorzugsweise schlitzartig und/oder kreisartig und erstrecken sich vorzugsweise über die komplette Länge und/oder Tiefe der Schubladenreling und/oder der Querreling und/oder der Zarge. Die Ausnehmungen sind vorzugsweise auf die Leuchtmittel bzw. die Leuchtdioden abgestimmt, so dass es wenig Streuverluste gibt. Die Schubladenreling und/oder die Querreling und/oder die Zarge kann auch aus einem Material sein, z.B. Quarzglas, welches durchlässig für das funktionale Licht ist, also für den UVC-Bereich und/oder den Infrarotbereich.

An den Schubladenseitenwänden und/oder den Seitenwandelementen und/oder an der Schubladenrückwand und/oder an dem Schubladenboden und/oder an der Schubladenfront ist das Leuchtmittel vorteilhafterweise direkt an der Oberfläche, welche in den Innenraum des Möbelkorpus und/oder des beweglichen Möbelteils ausgerichtet ist, angeordnet.

Die Schubladenseitenwände und/oder die Seitenwandelemnte und/oder die Schubladenrückwand und/oder der Schubladenboden und/oder die Schubladenfront kann eine Vertiefung für das Leuchtmittel aufweisen, wobei die Leuchtmittel vorzugsweise versenkt in der Vertiefung untergebracht sind.

Das Leuchtmittel kann auch an einer Bodenunterseite angeordnet sein, so dass eine Schublade, welche sich unterhalb der Bodenunterseite befindet, angestrahlt wird.

Die Schubladenreling ist vorzugsweise U-förmig ausgebildet. Weiter erstreckt sich die Schubladenreling vorzugsweise über die komplette Tiefenabmessung bzw. Länge der Schubladenseitenwände, insbesondere über 80% der Tiefenabmessung bzw. Länge der Schubladenseitenwände.

Die Querreling verbindet bevorzugterweise die beiden Schubladenseitenwände miteinander, wobei die Querreling senkrecht zur Tiefenabmessung bzw. Länge der Schubladenseitenwände angeordnet ist. Die Querreling kann auch die Schubladenfront und die Schubladenrückwand miteinander verbinden. Die Querreling ist vorzugsweise quaderförmig oder U-förmig, wobei das Leuchtmittel bzw. das Leuchtdiodenband zum Innenraum der Schublade ausgerichtet ist. Ist die Querreling U-förmig ausgebildet, zeigt vorzugsweise die offene Seite der Querreling in Richtung des Innenraums der Schublade, so dass das Leuchtmittel direkt in den Innenraum der Schublade einstrahlen kann. Ist die Querreling quaderförmig, weist die Seite, die zum Innenraum der Schublade ausgerichtet ist, vorzugsweise schlitzartige und/oder kreisförmige Ausnehmungen auf, damit das Leuchtmittel direkt in den Innenraum der Schublade strahlen kann.

Auch von Vorteil ist, dass am Möbelkorpus und/oder dem beweglichen Möbelteil zumindest ein lichtreflektierendes Element angeordnet ist, wobei das funktionale Licht von dem Leuchtmittel auf das lichtreflektierende Element trifft und das lichtreflektierende Element das funktionale Licht zumindest teilweise reflektiert, so dass das reflektierte funktionale Licht zumindest eine Oberflächen des Möbelkorpus und/oder des beweglichen Möbelteils bestrahlt.

Vorzugsweise sind mehrere lichtreflektierende Elemente am Möbelkorpus und/oder dem beweglichen Möbelteil angeordnet. Beispielsweise können die lichtreflektierenden Elemente in mehreren Reihen an einer Oberfläche des Möbelkorpus angeordnet werden. Des weiteren werden vorteilhafterweise die lichtreflektierenden Elemente so am Möbelkorpus und/oder dem beweglichen Möbelteil angeordnet, dass die Oberfläche des Möbelkorpus und/oder des beweglichen Möbelteils, welche zum Innenraum des Möbels ausgerichtet ist, mit funktionalen Licht bestrahlt wird.

Die lichtreflektierenden Elemente sind vorzugsweise plattenartig ausgebildet. Vorzugsweise haben die lichtreflektierenden Elemente eine flache und/oder eine gewölbte Außenkontur. Die Wölbung der Außenkontur der lichtreflektierenden Elemente kann konkav oder konvex sein. Eine weitere Möglichkeit besteht darin, dass die Flächen des beweglichen Möbelteils und/oder des Möbelkorpus aus einem lichtreflektierendem Material sind.

Es ist überdies vorteilhaft, dass am Möbelkorpus und/oder am beweglichen Möbelteil lichtreflektierende Elemente angeordnet sind, so dass das funktionale Licht alle Oberflächen des Möbelkorpus und/oder des beweglichen Möbelteils bestrahlt.

Vorzugsweise sind die lichtreflektierenden Element im Möbelkorpus und/oder am beweglichen Möbelteil so angeordnet, dass mittels der lichtreflektierenden Element sowohl die Oberflächen des Möbels, welche zum Innenraum des Möbels ausgerichtet sind, als auch die Oberflächen von Gegenständen im Innenraum des Möbels mit funktionalen Licht bestrahlt werden. Damit können die Oberflächen der Gegenstände im Innenraum des Möbels als die Oberflächen im Innenraum des Möbels entkeimt und/oder erwärmt werden. Bevorzugterweise werden alle Oberflächen der Gegenstände und des Möbels im Innenraum des Möbels durch das funktionale Licht bzw. das reflektierte funktionale Licht entkeimt und/oder erwärmt.

Ebenfalls von Vorteil ist, dass im Möbelkorpus und/oder im beweglichen Möbelteil an unterschiedlichen Wänden des Möbelkorpus und/oder des beweglichen Möbelteils Leuchtmittel angeordnet sind, so dass die Leuchtmittel sowohl Oberflächen im Innenraum des Möbelkorpus und/oder beweglichen Möbelteil als auch Gegenstände im Innenraum des Möbelkorpus und/oder des beweglichen Möbelteil mit funktionalen Licht bestrahlt werden.

Am Möbelkorpus können die Leuchtmittel an den Korpusseitenwänden, an der Korpusrückwand, am Korpusboden und/oder der Korpusoberseite angeordnet sein. Falls im Möbelkorpus, Einlegeböden angeordnet sind, können auch diese mit Leuchtmitteln ausgestattet werden. Bei einem Einlegeboden kann auch an einer Hauptfläche oder beiden Hauptflächen, also die Flächen welche in Richtung Boden und Oberseite ausgerichtet sind, Leuchtmittel angeordnet sein.

Wenn das bewegliche Möbelteil z.B. eine Oberklappe oder eine Schranktür ist, kann das Leuchtmittel dort angeordnet sein. Die Leuchtmittel können beim beweglichen Möbelteil, wenn das bewegliche Möbelteil eine Schublade ist, an einer Schubladenseitenwand, an einer Schubladenrückwand, an einer Schubladenfront, an einer Schubladenreling, an einer Zarge, an einem Seitenwandelement, an einer Querreling und/oder an einem Schubladenboden angeordnet sein. Die Leuchtmittel können auch an einer Unterseite des Schubladenbodens angeordnet sein, so dass ein Innenraum, einer unterhalb des Schubladenbodens befindliche Schublade, mit funktionalen Licht beleuchtet wird.

### Figurenbeschreibung

Weitere Merkmale und Vorteile der Erfindung sind anhand von in den Figuren schematisch dargestellten Ausführungsbeispielen näher erläutert.

Im Einzelnen zeigt:
- Fig. 1: eine perspektivische Ansicht von schräg oben auf ein Möbel mit einem Möbelkorpus und einer Schublade, wobei die Schublade im vollständig geöffneten Zustand dargestellt ist,
- Fig. 2: eine perspektivische Ansicht von schräg oben auf eine Schublade mit einer Schubladen-Reling,
- Fig. 3: eine perspektivische Ansicht schräg von der Seite auf einen Oberschrank mit einem Möbelkorpus und einer Oberklappe, wobei die Oberklappe im vollständig geöffneten Zustand dargestellt ist,
- Fig. 4: eine perspektivische Ansicht schräg von der Seite auf einen Schrank mit einem Möbelkorpus und einer Schranktür, wobei die Schranktür im geöffneten Zustand dargestellt ist,
- Fig. 5: ein schematisches Schaubild einer Beleuchtungsanordnung,
- Fig. 6: ein alternatives schematisches Schaubild der Beleuchtungsanordnung.

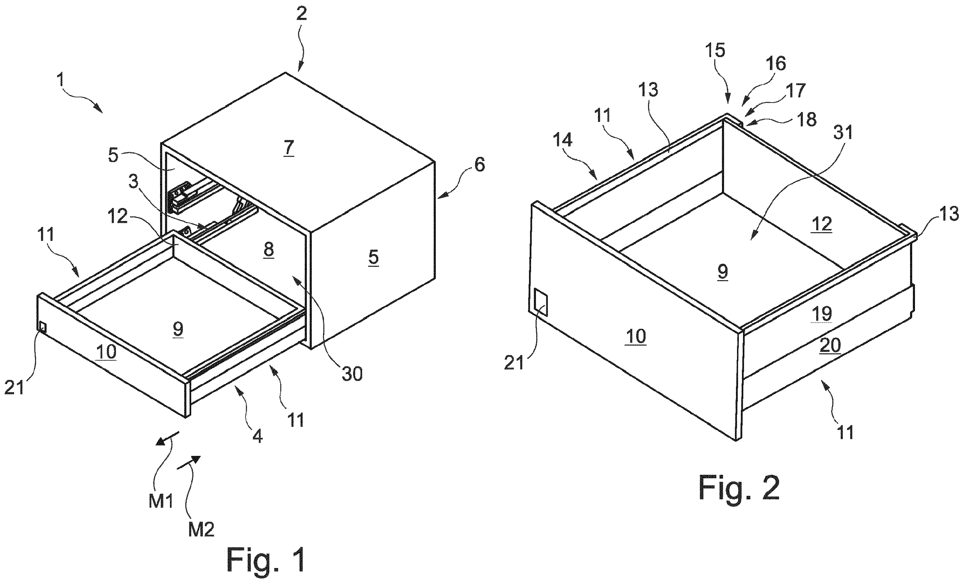

Ein erfindungsgemäßes Möbel 1 mit einem vorne offenen kastenförmigen Möbelkorpus 2 und einer über Führungsmittel 3 beweglich geführte Schublade 4 ist in Figur 1 dargestellt. Die Schublade 4 ist in Figur 1 in einem geöffneten Zustand dargestellt.

Der Möbelkorpus 2 umfasst zwei sich gegenüberliegende Korpusseitenwände 5, eine Korpusrückwand 6, einen Korpusoberseite 7 und einen Korpusboden 8, welche den Innenraum 30 des Möbelkorpus 2 umgeben.

Die Schublade 4 umfasst einen Schubladenboden 9, eine Schubladenfront 10, zwei gegenüberliegende Schubladenseitenwände 11 und eine Schubladenrückwand 12, welche einen Innenraum 31 der Schublade 4 umgeben. Die Schubladenseitenwand 11 umfasst vorzugsweise ein Seitenwandelement 19 und eine Zarge 20. Für die Führung der Schublade 4 sind zwei gleichwirkende Führungsmittel 3 jeweils zwischen einer Schubladenseitenwand 11 und einer dazugehörigen Korpusseitenwand 5 vorhanden. Die Schublade 4 ist über die Führungsmittel 3, beispielsweise zwei gleichartige Teil- oder Vollauszüge, am Möbelkorpus 2 in eine Öffnungsrichtung M1 und in eine Schließrichtung M2 verschiebbar gelagert.

Die Schublade 4, welche gemäß der Fig. 1 und 2 den gleichen Grundaufbau aufweist, ist in Fig. 2 zur Schublade 4 in Fig. 1 in anderen Proportionen dargestellt.

Die Schublade 4 weist eine Schubladenreling 13 auf, die oberhalb der Schubladenseitenwand 11 bzw. des Seitenwandelements 19 vorhanden ist. Dabei ist vorzugsweise ein Leuchtmittel 14 einer Beleuchtungsanordnung 15 an der Schubladenreling 13 bzw. jeweils ein Leuchtmittel 14 an beiden Schubladenreling 13 angeordnet. Die Beleuchtungsanordnung 15 umfasst neben den Leuchtmittel 14, eine Kontrolleinheit 16, eine Sensoreinheit 17 und einen Energiespeicher 18. Der Energiespeicher 18 ist bevorzugterweise an einer Rückseite der Schubladenrückwand 12 angeordnet.

Die Kontrolleinheit 16 und/oder die Sensoreinheit 17 ist zumindest im Wesentlichen im Inneren des Energiespeichers 18 und/oder der Leuchtmittel 14 untergebracht (in den Figuren nicht ersichtlich). Die Sensoreinheit 17 ist vorzugsweise so angeordnet, dass diese die Bewegung der Schublade 4 misst. Das Leuchtmittel 14 ist vorzugsweise aus mehreren Leuchtdioden gebildet (nicht dargestellt), welche vorzugsweise an einem Band angeordnet sind. Die Leuchtdioden sind bevorzugterweise hintereinander und versetzt zueinander am Band angeordnet. Vorteilhafterweise erstrecken sich die Leuchtmittel 14 über die komplette Länge der Schubladenrelinge 13.

Die Leuchtmittel 14 können auch an unterschiedlichen Flächen bzw. an mehreren Flächen der Schublade 4, die zum Innenraum 31 der Schublade 4 ausgerichtet sind, angeordnet sein, wie z.B. der Zarge 20, dem Seitenwandelement 19, der Schubladenrückwand 12, dem Schubladenboden 9, der Schubladenseitenwand 11 und/oder der Schubladenfront 10. Auch an Fläche des Möbelkorpus, welche zum Innenraum 30 des Möbelkorpus 2 ausgerichtet sind, können die Leuchtmittel 14 angeordnet sein, wie beispielsweise an den Korpusseitenwänden 5, der Korpusrückwand 6, dem Korpusboden 8 und/oder der Korpusoberseite 7. Auch können die Leuchtmittel an Flächen des Möbelkorpus 2 und an Flächen der Schublade 4 angeordnet sein.

Weiter ist vorzugsweise an der außenliegenden Fläche der Schubladenfront 10 ein Schalter 21, vorzugsweise ein mechanischer Schalter, angeordnet, welcher durch eine Bedienperson aktiviert werden kann. Der Schalter 21 kann auch an einer für die Bedienperson zugängliche Fläche des Möbelkorpus 2 angeordnet sein, wie z.B. der außenliegenden Fläche der Korpusseitenwand 5. Der Schalter 21 ist bevorzugterweise ein Druckschalter oder ein Funkschalter.

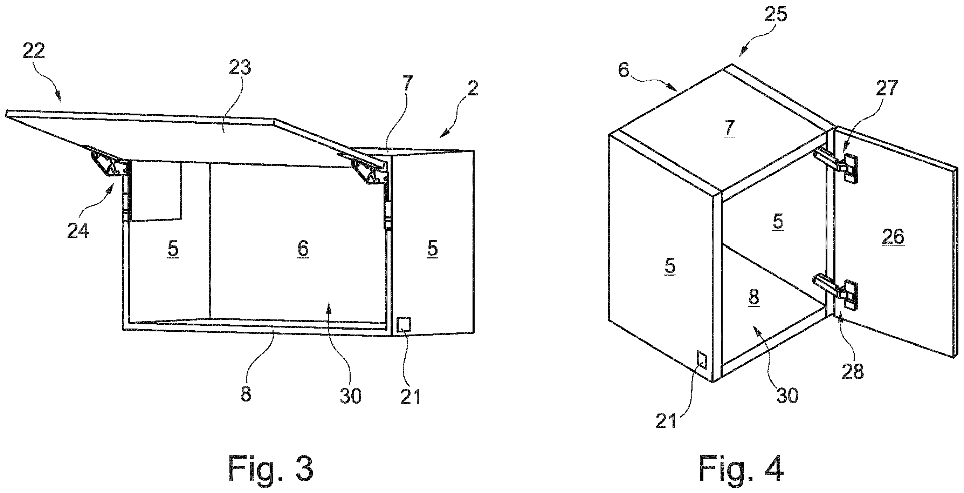

Die Figur 3 zeigt ein Möbel 1 bzw. einen Oberschrank 22 mit einem kastenförmigen Möbelkorpus 2 und einem daran aufgenommenen beweglichen Möbelteil, das als eine plattenartige Oberklappe 23 ausgebildet ist. Dabei ist in Figur 3 die Offenstellung der Oberklappe 23 gezeigt. Der Möbelkorpus 2 umfasst zwei gegenüberliegende, aufrechte Korpusseitenwände 5, die unten mit einem Korpusboden 8 und oben mit einer Korpusoberseite 7 verbunden sind. Rückseitig ist der Möbelkorpus 2 von einer Korpusrückwand 6 verschlossen. Zur Bewegung der Oberklappe 23, um eine horizontale Schwenkachse relativ zum Möbelkorpus 2 aus der in Fig. 3 gezeigten Offenstellung in eine zum Möbelkorpus 2 frontseitig heranbewegte Schließstellung (nicht gezeigt), ist ein Oberklappenbeschlag 24 ausgestaltet. Es sind vorzugsweise zwei Oberklappenbeschläge 24 an jeweils einer Korpusseitenwand 5 spiegelsymmetrisch zueinander ausgebildet.

Bevorzugsterweise ist in einer oberen Ecke der Korpusrückwand 6 der Energiespeicher 18 angeordnet, wobei der Energiespeicher 18 bevorzugterweise mit einer Seitenkanten an einer Korpusseitenwand 5 und einer anderen Seitenkante an der Koprusoberseite 7 anliegt.

Der Schalter 21 ist vorteilhafterweise an einer außenliegenden Fläche des Möbels, bevorzugterweise der Korpusseitenwand 5, angebracht, so dass der Schalter 21 einfach zugänglich für die Bedienperson ist.

Das Leuchtmittel 14 bzw. die Leuchtmittel 14 sind vorzugsweise an Flächen des Möbelkorpus 2, welche zum Innenraum 30 des Möbelkorpus ausgerichtet sind, angeordnet, wie z.B. an den Korpusseitenwänden 5, der Korpusrückwand 6, der Korpusoberseite 7 und der Korpusboden 8. Die Leuchtmittel 14 sind dabei bevorzugterweise direkt an den flachen, ebenen Oberfläche oder in Vertiefungen an diesen Flächen angebracht.

Die Figur 4 zeigt ein als Schrank 25 ausgebildetes Möbel 1. Der Schrank 25 ist stark vereinfacht dargestellt, insbesondere ohne mögliche im Innenraum 30 des Möbelkorpus 2 angeordnete Einlegeböden, wie beispielsweise Regalfächer. Am Schrank 25 ist ein als Schranktür 26 ausgebildetes bewegliches Möbelteil schwenkbar über z.B. zwei identische Möbelscharniere 27 und 28 aufgenommen. Die Möbelscharniere 27, 28 weisen z.B. jeweils genau eine Gelenkachse auf, welche senkrecht steht und um welche die Schranktüre 26 in eine Schließrichtung einschwenkbar und in eine entgegengesetzte Richtung aufschwenkbar ist. Die Schranktüre 26 ist im Bereich einer korpusseitigen Schmal- bzw. Stirnseite demgemäß am Möbelkorpus 2 über das obere Möbelscharnier 27 und das untere Möbelscharnier 28 an einer Korpusseitenwand 5 gelenkig bewegbar aufgenommen.

Ein Möbelkorpus 2 des Schranks 25 ist im wesentlichen quaderförmig mit einer offenen Frontseite, einer Korpusrückwand 6, einem Korpusboden 8, einer Korpusoberseite 7 und zwei Korpusseitenwänden 5.

Auch bei dem Möbelschrank 25 ist vorzugsweise der Energiespeicher 14 (nicht gezeigt) an einer oberen Ecke der Korpusrückwand 6 angeordnet, wobei Kanten des Energiespeichers 14 an einer Korpusseitenwand 5 und der Korpusoberseite 7 anliegen.

Die Leuchtmittel 14 sind, wie zuvor beim Oberschrank 22, vorzugsweise an den Flächen der Korpusseitenwände 5, der Korpusrückwand 6, der Korpusoberseite 7 und/oder des Korpusboden 8 angeordnet, welche zum Innenraum 30 des Möbelkorpus 2 ausgerichtet sind. Auch kann das Leuchtmittel 14 an der Schranktüre 26 angeordnet sein, welche zum Innenraum 30 des Möbelkorpus 2 ausgerichtet ist. Die Kontrolleinheit 16 ist, wie beim Möbel 1, auch beim Oberschrank 22 oder dem Schrank 25 vorzugsweise in dem Energiespeicher 18 bzw. dem Gehäuse des Energiespeichers 18 aufgenommen.

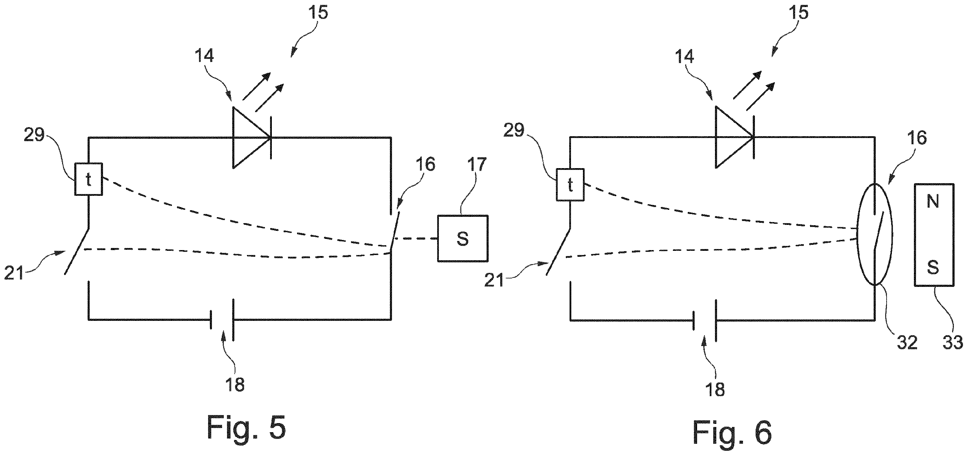

Es ist in Figur 5 schematisch der Aufbau der Beleuchtungsanordung dargestellt. Die Beleuchtungsanodnung 15 umfasst einen Energiespeicher 18, eine Kontrolleinheit 16, eine Sensoreinheit 17, ein Leuchtmittel 14 bzw. Leuchtdiode, ein Zeitglied 29 und einen Schalter 21.

Ist das bewegliche Möbelteil in einem geschlossenen Zustand, kommuniziert die Sensoreinheit 17 dies an die Kontrolleinheit 16, welche den Schalter der Kontrolleinheit 16 schließt. Möchte nun beim geschlossenen Zustand des beweglichen Möbelteils die Bedienperson die Leuchtmittel 14 aktivieren, so schließt die Bedienperson manuell den Schalter 21, z.B. durch Drücken oder durch ein Funksignal, so dass der Stromkreis geschlossen ist und die Leuchtmittel 14 eingeschaltet werden. Die Leuchtdauer der Leuchtmittel 14, also die Zeit, die vergeht nach dem Einschalten bis zum selbsttätigen Ausschalten, wird von dem Zeitglied 29 vorgegeben, wobei die von dem Zeitglied 29 vorgebbare Zeitdauer nach Aktivieren des Schalters 21 beginnt. Nach Ablauf der vorgebaren Zeitdauer durch das Zeitglied 29 wird vorzugsweise der Schalter 21 wieder geöffnet. Beispielsweise kommuniziert die Kontrolleinheit 16 mit dem Zeitglied 29 und dem Schalter 21 und kann somit den Schalter 21 nach der abgelaufenen Zeit schalten bzw. öffnen. Wird vor Ablauf der vorgebaren Zeitdauer bzw. bei eingeschalteten Leuchtmittel 14 das bewegliche Möbelteil geöffnet, so wird dies von der Sensoreinheit 17 erfasst und an die Kontrolleinheit 16 kommuniziert, so dass die Kontrolleinheit 16 den Schalter der Kontrolleinheit 16 öffnet und der Stromfluss zum Leuchtmittel 14 unterbrochen wird.

Die Kontrolleinheit 16 kann auch so ausgebildet sein, dass diese die Bewegungszustände des beweglichen Möbelteils wahrnimmt (s. Fig. 6). Beispielsweise kann die Kontrolleinheit 16 eine Reedeinheit, welche einen Reedschalter 32 und einen Magneten 33 umfasst (s. Fig. 6), umfassen. Beim Annähern des Reedschalters 32 an den Magneten 33 schließt der Reedschalter 32. Dabei ist bei einem Möbel 1 mit Schublade 4 bevorzugterweise der Reedschalter 32 an der Schublade 4, insbesondere an der Rückseite der Schubladenrückwand 12 angeordnet und der Magnet 33 an der Korpusrückwand 6, an der zur Schublade 4 liegenden Fläche, so dass beim Annähern der Schublade 4 an die Korpusrückwand 6 der Magnet 33 den Reedschalter 32 schaltet.

Weiter sind bevorzugterweise alle Elemente der Beleuchtungsanordnung 15, die am Stromkreis angeschlossen sind, an der Schublade 4 angeordnet.

Bei einem Oberschrank 22 (Fig. 3) oder einem Schrank 25 (Fig. 4) sind die Elemente der Beleuchtungsanordnung 15, die am Stromkreis angeschlossen sind, vorzugsweise am Möbelkorpus 2 angeordnet.

### Bezugszeichenliste

- 1: Möbel
- 2: Möbelkorpus
- 3: Führungsmittel
- 4: Schublade
- 5: Korpusseitenwand
- 6: Korpusrückwand
- 7: Korpusoberseite
- 8: Korpusboden
- 9: Schubladenboden
- 10: Schubladenfront
- 11: Schubladenseitenwand
- 12: Schubladenrückwand
- 13: Schubladenreling
- 14: Leuchtmittel
- 15: Beleuchtungsanordnung
- 16: Kontrolleinheit
- 17: Sensoreinheit
- 18: Energiespeicher
- 19: Seitenwandelement
- 20: Zarge
- 21: Schalter
- 22: Oberschrank
- 23: Oberklappe
- 24: Oberklappenbeschlag
- 25: Schrank
- 26: Schranktür
- 27: Möbelscharnier
- 28: Möbelscharnier
- 29: Zeitglied
- 30: Innenraum
- 31: Innenraum
- 32: Reedschalter
- 33: Magnet

## Patentansprüche

1. Möbel (1) mit einem Möbelkorpus (2) und einem beweglichen Möbelteil (4, 23, 26), wobei das bewegliche Möbelteil (4, 23, 26) relativ zum Möbelkorpus (2) beweglich ist, wobei am Möbelkorpus (2) und/oder an dem beweglichen Möbelteil (4, 23, 26) eine Beleuchtungsanordnung (15) angeordnet ist, wobei die Beleuchtungsanordnung (15) ein Leuchtmittel (14) umfasst, **dadurch gekennzeichnet, dass** das Leuchtmittel (14) ein funktionales Licht in einen Innenraum (30) des Möbelkorpus (2) und/oder in einen Innenraum (31) des beweglichen Möbelteils (4) abstrahlt, wobei das funktionale Licht Luft und/oder Oberflächen und/oder Gegenständen in einen Innenraum (30) des Möbelkorpus (2) und/oder einen Innenraum (31) des beweglichen Möbelteils (4) entkeimt und/oder erwärmt.

2. Möbel nach Anspruch 1, **dadurch gekennzeichnet, dass** das funktionale Licht, welches das Leuchtmittel (14) abstrahlt, im UVC-Bereich liegt, insbesondere eine Wellenlänge zwischen 200-270 nm aufweist.

3. Möbel nach Anspruch 1, **dadurch gekennzeichnet, dass** das funktionale Licht des Leuchtmittels (14) im Infrarotbereich liegt.

4. Möbel nach einem der vorangegangenen Ansprüche, **dadurch gekennzeichnet, dass** die Beleuchtungseinrichtung (15) eine Sensoreinheit (17) und eine Kontrolleinheit (16) umfasst, wobei die Sensoreinheit (17) einen Bewegungszustand des beweglichen Möbelteils (4, 23, 26) erfasst und wobei die Kontrolleinheit (16) ausgebildet ist, abhängig von einem mit der Sensoreinheit (17) erfassten Bewegungszustand des beweglichen Möbelteils(4, 23, 26), das Leuchtmittel (14) ein- und/oder auszuschalten, so dass das Leuchtmittel (14) ausschließlich bei einem geschlossen Zustand des Möbels (1) einschaltbar ist.

5. Möbel nach einem der vorangegangenen Ansprüche, **dadurch gekennzeichnet, dass** die Kontrolleinheit (16) so ausgebildet ist, dass das Leuchtmittel (14), nur im geschlossenen Zustand des Möbels (1), von einer Bedienperson aktivierbar ist.

6. Möbel nach einem der vorangegangenen Ansprüche, **dadurch gekennzeichnet, dass** die Kontrolleinheit (16) ausgebildet ist, das Leuchtmittel (14) nach dem Aktivieren durch eine Bedienperson, für eine vorgebbare Zeitdauer einzuschalten.

7. Möbel nach einem der vorangegangenen Ansprüche, **dadurch gekennzeichnet, dass** die Beleuchtungsanordnung (15) einen Energiespeicher (18) umfasst, wobei der Energiespeicher (18) das Leuchtmittel (14) und/oder die Kontrolleinheit (16) mit elektrischer Energie versorgt.

8. Möbel nach einem der vorangegangenen Ansprüche, **dadurch gekennzeichnet, dass** die Kontrolleinheit (16) ausgebildet ist, bei einer durch die Sensoreinheit erfassten Öffnungsbewegung des beweglichen Möbelteils (4, 23, 26) das Leuchtmittel (14) auszuschalten.

9. Möbel nach einem der vorangegangenen Ansprüche, **dadurch gekennzeichnet, dass** die Kontrolleinheit (16) ausgebildet ist, einen Sperrmechanismus zu bedienen, wobei der Sperrmechanismus derart ausgebildet ist, das bewegliche Möbelteil (4, 23, 26), wenn das Leuchtmittel (14) eingeschaltet ist, zu versperren und bei ausgeschalteten Leuchtmittel (14) das bewegliche Möbelteil (4, 23, 26) wieder zu entsperren.

10. Möbel nach einem der vorangegangenen Ansprüche, **dadurch gekennzeichnet, dass** das bewegliche Möbelteil (4, 23, 26) eine Schublade (4) ist, wobei das Leuchtmittel (14) an einer Schubladenreling (13) und/oder Querreling und/oder an einer Schubladenseitenwand (11) und/oder an einer Zarge (20) und/oder an einem Seitenwandelement (19) und/oder an einer Schubladenrückwand (12) und/oder an einem Schubladenboden (9) und/oder an einer Schubladenfront (10) angeordnet ist

11. Möbel nach einem der vorangegangenen Ansprüche, **dadurch gekennzeichnet, dass** im Möbelkorpus (2) und/oder dem beweglichen Möbelteil (4, 23, 26) zumindest ein lichtreflektierendes Element angeordnet ist, wobei das funktionale Licht von dem Leuchtmittel (14) auf das lichtreflektierende Element trifft und das lichtreflektierende Element das funktionale Licht reflektiert, so dass das funktionale Licht zumindest eine Oberflächen des Möbelkorpus (2) und/oder des beweglichen Möbelteils (4, 23, 26) bestrahlt.

12. Möbel nach einem der vorangegangenen Ansprüche, **dadurch gekennzeichnet, dass** im Möbelkorpus (2) und/oder dem beweglichen Möbelteil (4, 23, 26) lichtreflektierende Elemente angeordnet sind, so dass das funktionale Licht alle Oberflächen des Möbelkorpus (2) und/oder des beweglichen Möbelteils (4, 23, 26) bestrahlt.

13. Möbel nach einem der vorangegangenen Ansprüche, **dadurch gekennzeichnet, dass** im Möbelkorpus (2) und/oder im beweglichen Möbelteil (4, 23, 26) an unterschiedlichen Wänden des Möbelkorpus (2) und/oder des beweglichen Möbelteils (4, 23, 26) Leuchtmittel (14) angeordnet sind, so dass die Leuchtmittel (14) sowohl Oberflächen im Innenraum des Möbelkorpus (2) und/oder beweglichen Möbelteil (4, 23, 26) als auch Gegenstände im Innenraum des Möbelkorpus (2) und/oder des beweglichen Möbelteils (4, 23, 26) mit funktionalen Licht bestrahlen.
